# EUROPEAN PATENT SPECIFICATION

(11) **EP 3 258 987 B1**
(45) Date of publication and mention of the grant of the patent: **26.09.2018**
(21) Application number: 15718762.6
(22) Date of filing: 19.02.2015
(51) Int. Cl.: A61L 31/04, A61L 31/14, A61M 5/38, A61M 5/14, A61M 5/168, A61M 39/24, A61M 5/165, A61M 39/22

(54) **DEVICE FOR ADMINISTRATION OF FLUID MEDICAMENTS**
VORRICHTUNG ZUR VERABREICHUNG VON FLÜSSIGEN MEDIKAMENTEN
DISPOSITIF D'ADMINISTRATION DE MÉDICAMENTS FLUIDES

(43) Date of publication of application: 27.12.2017
(73) Proprietor: UMC Utrecht Holding B.V., 3584 CM Utrecht (NL)
(72) Inventor: RIPHAGEN, Brechtje, NL-3512 TX Utrecht (NL); EVERS, Lucas Alphonsus Maria, NL-3707 CL Zeist (NL); MIJERS, Jan Willem Marinus, NL-2014 AL Haarlem (NL); VAN ROSSUM, Lex Alexander Franciscus, NL-8102 EA Raalte (NL)
(74) Representative: Demski, Siegfried
(86) International application number: PCT/EP2015/000365
(87) International publication number: WO 2016/131463

(56) References cited:
- WO-A2-2014/047626
- US-A1- 2007 161 970

## Description

The present invention relates to a device for the administration of fluids to a human or animal body, comprising a housing with a first inlet, at least one second inlet, one outlet and a collecting point, wherein the first inlet communicates with the collecting point through a first channel and the outlet communicates with the collecting point.

Such devices are known from prior art and serve the purpose of administering medicaments to humans, in particular infants, or, in some cases, to animals. There are some medicaments which need to be administered with a precise dose, as they have toxic properties and negative side effects. A patient should be exposed to such medicaments only as much as is necessary. Too high a dosage of such medicaments can cause serious damage to a patient and can even cause the death of the same. On the other hand, a dosage too low leads to an insufficient treatment of the respective medical indication, while nevertheless exposing the patient to such medicaments, and thus, causing negative side effects.

An apparatus for administering multiple fluid medicaments to a patient is known from US 3,941,126 A. The apparatus allows for an independent adjustment of the dilution of the individual medicaments fed to it. The dilutor is fed from a storage vessel into cylindrical containers through valves, where the amount of the dilutor can be adjusted for each container. Subsequently, the medicaments are added to the individual containers with the aid of syringes. By means of additional valves the diluted medicaments flow into a drip chamber through separate tubes where they converge. The mixture of diluted medicaments then flows to a point of administration, e.g. an infusion needle, through another tube.

US 2009/0137951 A1 discloses an apparatus for transferring several fluid medicaments into a tube with several channels. The tube ends in a mixing chamber arranged in the proximity of a patient. The medicaments converge within the mixing chamber. The medicament mixture then flows from the mixing chamber to an administration point that can be embodied as an infusion needle, e.g.. Thus, owing to the said tube featuring several channels, the medicaments converge shortly before the administration point.

US application 2007/0161970 A1 discloses a device for the feeding of medicaments. Basically, only two supplies are fed to a valve, which features a supply to the patients on the output side. When the administration of further medicaments is necessary, the additional medicament may be fed to one of the two supplies by use of an additional valve. However, the valve controller does not allow for a previous mixture of a certain amount of medicaments.

A manifold valve is known from WO 2014/047626 A2, which allows for the possibility of an automatic dosage of medicaments. The mixture of several medicaments in a corresponding device is not disclosed.

It is an object of the present invention to provide a device for administering fluid medicaments to a patient with a more precise dosage with respect to devices known from prior art, especially with low flow rates in the range of 0.5 ml/h to 20 ml/h.

This is achieved by a device with the features of claim 1. The device according to the invention is characterized in that the second inlet communicates with the collecting point or an indicator chamber through a second channel, in which a valve is arranged, while said valve is adjustable between a first position and a second position, wherein the second channel communicates with the indicator chamber in the first position of the valve, and the second channel communicates with the collecting point in the second position of the valve.

First of all, it should be noted that the expressions fluid and medicament are used interchangeably throughout the present description of the invention, since the medicaments that are to be administered to a patient by means of the device according to the invention are in the form of a fluid, i.e. fluid substances or solutions of originally solid substances. In the latter case, the solvent is in most cases water, which might already contain sodium chloride. Furthermore, a solution without a physiological effect on a patient, in particular a sodium chloride solution, can be fed to one of the inlets serving as a transportation medium for the medicaments which are to be administered and which are fed to the second inlet. Furthermore, a nutritive solution can be fed to the device. The fluids fed to the first and second inlets are collected in the collecting point that is therefore denominated as such.

In addition to the collecting point, the device comprises an indicator chamber whose purpose is to indicate, in particular making visible, that the flow path between the second inlet and the valve, including the volume of the valve which is exposed to the fluid fed to the second inlet, is filled with fluid. Once this is ensured, the valve is adjusted from its first position to its second position, in which the second channel communicates with the collecting point. Since the second channel and the fluid guiding volume of the valve is already filled with fluid, converging of the fluid fed to the second inlet and the fluid fed to the first inlet occurs immediately after the valve is switched in its second position, and thus, the delay between adjusting the valve and administering the mixture of fluids to a patient is minimized. This allows for a precise setting of the point in time at which the administration of medicaments starts and therefore, for a more precise calculation of the dose (dose = [volume of medicament / time unit] x administration period) actually administered to a patient. For these reasons, the indicator chamber is an essential feature of the device according to the invention.

When employing the device according to the invention, tubes are connected to the first inlet, to the at least one second inlet and to the outlet, respectively. The tube connected to the outlet is connected with an infusion needle on its proximal end. Then, pumps are activated feeding a first fluid to the first inlet through a first tube and a second fluid to the second inlet through a second tube. This first fluid is generally a sodium chloride solution or a nutritive solution. The second fluid is regularly a medicament. The valve of the device is in its first position and provides thus a fluid connection between the second inlet and the indicator chamber via the second channel. After a short while, the indicator chamber is filled with the second fluid which is at least detectable electronically, e.g. by means of a detector, but preferably visible. In the next step, the valve is adjusted to its second position, in which state the valve provides a fluid connection between the second inlet and the collecting point via the second channel. Now, the second fluid being fed through the second channel and the first fluid being fed through the first channel converge at the collecting point. The mixture of the fluids is then fed to the outlet communicating with the collecting point. If administration of the second fluid, normally a medicament, shall end, the valve is switched back to its first position so as to block the fluid connection between the second inlet and the collecting point. On the other hand, the fluid connection between the first inlet and the collecting point is still existent, and the first fluid, normally a sodium chloride solution or a nutritive solution, is still administered to the patient. The valve can later be adjusted to its second position again in order to further administer the second fluid to the patient if desired or necessary. In the latter case, there is again almost no delay between adjusting the valve and the administration of the second fluid, since the flow path is already filled with the second fluid.
It is pointed out, that a device comprising one first inlet and one second inlet is merely a basic embodiment of the device according to the invention. Preferably, there are two second inlets and, correspondingly, two second channels, two valves and two indicator chambers. However, more than two second inlets and, correspondingly, more than two second channels, two second valves and two indicator chambers can be provided as well.

Furthermore, according to the invention, the indicator chamber further comprises an indicator element which changes its color in case of fluid contact.

Further advantageous embodiments of the device according to the invention result from the sub-claims.
According to such an advantageous embodiment, the indicator chamber and/or the housing is at least in part transparent or translucent for visible electromagnetic radiation, i.e. for visible light. This feature allows for a simple detection of whether the indicator chamber is filled with the fluid fed to the second inlet, namely by means of a human eye.

According to a further embodiment of the invention, the indicator chamber comprises a hydrophobic filter element. Obviously, there can be both a hydrophobic filter element and an indicator element arranged within the indicator chamber. A hydrophobic filter element allows air or gas bubbles to escape, which must not enter a patient's blood circuit. This is due to the well-known fact that air or other gases within the blood circuit can lead to a failure of the heard or other organs, since all organs need a blood supply for the supply with oxygen and nutritive substances. Blood also serves for temperature control of the organs and for the transport of products of metabolism. Correspondingly, the indicator chamber must comprise a wall with at least one opening communicating with the ambience air of the device and against which the hydrophobic filter element abuts in order to allow gas bubbles to escape.
A wall or a section of a wall of the indicator chamber, that is transparent for visible light, might already allow for the observation whether a fluid is present in the indicator chamber. However, according to the invention, there is an indicator element arranged in the indicator chamber, which changes its color in case of fluid contact, such that the presence of a fluid in the indicator chamber is easily observable by the human eye and accordingly without further technical equipment. Of course, being observable by the human eye requires that a wall or a section of a wall of the indicator chamber is transparent or translucent for visible light.
The indicator element can be made of a sintered plastic material. Such a sintered plastic material combines the functionalities of a hydrophobic filter element on the one hand, and of an indicator element on the other hand. Such sintered plastic materials are porous before fluid contact and change their density in case of fluid contact, whereat the small channels of the previously porous plastic material get sealed. This might not be true for all plastic materials, but there are some plastic materials available which have this property in the sintered state. By means of at least one additive, a color transition in case of fluid contact can be achieved as well, in which case the indicator element serves as hydrophobic filter and as color changing indicator. Due to this synergy effect, the embodiment just described is the particularly preferred embodiment of the device according to the invention. A suitable plastic material having said property, i.e. the said synergy effect, is sintered polyethylene (PEL), which is already available on the market.

As is the case with the hydrophobic filter element, the indicator element with such a property abuts on a wall of the indicator chamber with at least one opening communicating with the ambience air of the device, in order to allow gas bubbles to escape from the indicator chamber and thus, from the fluid fed to the second inlet.

Furthermore, it can be provided that an inlet opening of the indicator chamber is covered by an elastic sealing element acting as a check valve which is closed in a pressureless state, whereat the term pressureless refers to atmospheric pressure, i.e. pressure not exceeding atmospheric pressure. The sealing element serves as a check valve and can be provided in addition to check valves arranged in the first or second channel or without the latter check valves. It covers the inlet opening of the indicator chamber, extends marginally over the circumference of the inlet opening and can be held in position by a support which contacts the sealing element in its center, for instance, and exerts an initial tension on the sealing element such that the outer edge of the sealing element acts as sealing lip being raisable in only one direction.

Such support can be realized in that the indicator chamber comprises at least one insertion element which holds the sealing element in its position by contacting the sealing element and/or which receives the hydrophobic filter element and/or the indicator element. The insertion element can serve both purposes or only one of them. Correspondingly, a plurality of insertion elements can be provided, which serve different purposes and are possibly fixed together by frictional connections, tongue and groove connections, welding, gluing or in similar ways. For example, the wall of the indicator chamber with at least one opening can be embodied as a wall element that is fixed on the insertion element or on one or more of the insertion elements. The housing of the device might comprise a clearance forming the indicator chamber and into which the at least one insertion element is, together with the sealing element and/or the hydrophobic filter element and/or the indicator element, inserted and fixed to the circumferential surface of the clearance by means of, e.g., a frictional connection, a tongue and groove connection, welding, gluing or the like.

As an alternative to the indicator element, an indicator mechanism can be arranged within the indicator chamber, comprising at least one movable part which moves with respect to the indicator chamber, preferably out of the same, when the latter is filled with fluid, i.e. the pressure within the indicator chamber leads to a movement of the movable part. Such indicator mechanism can be embodied as a pin, which is the movable part, and a resilient member, in the form of a spring, e.g.. In this case, the pin moves against the restoring force of the resilient member due to the pressure within the indicator chamber, or the pin moves through the restoring force of the resilient member after the pressure within the indicator chamber has released a retainer that held the resilient member in a tensioned, i.e. compressed, state. Of course, a plurality of indicator mechanisms is conceivable by a skilled person and is considered to be within the scope of the present invention. The mentioned indicator mechanisms comprising a pin and a resilient member are merely chosen as an example. The indicator mechanism serves the same purpose as the indicator element referred to hereinbefore, namely, indicating wether the indicator chamber is filled with the fluid fed to the respective second channel.

The movable part can additionally feature a color mark, by means of which can be observed more easily that the movable part moved.

With a further embodiment of the device the second channel and/or the first channel comprise a filter chamber in which a hydrophilic filter member is arranged, wherein the filter member separates the filter chamber into to sub-chambers. This hydrophilic filter member allows for a removal of solid particles contained as contaminants in the medicament fed to the second inlet. Such contaminants can get into medicaments during the manufacturing process of the same, or by absorption from the air in non-decontaminated environments. However, they shall not reach the patient's blood, as agglomerates of such particles can block blood vessels or such particles can contain toxic ingredients like heavy metals. It is assumed that the hydrophilic filter members also contribute to an increased independency of flow rates within the single channels of the device, i.e. an adjustment of the flow rate within one channel has a lower effect on the flow rates within the other channels, since the hydrophilic filter members are flow resistances.

Furthermore, a hydrophobic filter member can be arranged in the filter chamber, which covers an opening of the filter chamber being in communication with the ambience air of the device. In this case, an opening is located in the filter chamber that communicates with the ambience air of the device, which means that the opening extends through the housing of the device. The opening is covered by the hydrophobic filter member allowing gas bubbles, in particular air bubbles, possibly contained in the fluid, which is fed to the second inlet, to escape. Air bubbles must be prevented from reaching the patient's blood circuit as set forth above.

The hydrophilic filter member and the hydrophobic filter member are preferably fixed to the filter chamber by welding, in particular ultra sonic welding, wherein the welding seam extends along the outer edge of the filter members. However, a fixation by means of gluing is also possible.

The hydrophilic filter member can be supported by protrusions extending from at least one wall of the filter chamber. The protrusions of this embodiment ensure that the hydrophilic filter member does not lose its flat shape. Moreover, the hydrophilic filter member can be fixed on some or all of the protrusions, in order to provide a stronger fixation of the hydrophilic filter member.

In order to prevent back flow of the fluids, a check valve can be arranged in the first channel and/or in the second channel. Preferably, one check valve is arranged in the first channel in the vicinity of the collecting point and one check valve is arranged in each second channel in the vicinity of the respective valve.

It is important that the dead volume of the device is comparatively small, since a larger dead volume leads to a longer delay between a switching of the valve and the administration of the fluid fed to the second inlet to a patient. It is assumed that the dead volume contributes to a dependency of flow rates, causing fluctuations of other flow rates if one flow rate is changed. It is assumed that a small dead volume minimizes this effect. The volume of the collecting point should therefore be small, which is why the denomination "collecting point" was chosen. However, the collecting point can be embodied as a collecting chamber with a larger dead volume due to design purposes, and in particular due to the fact that very small structural elements of the device lead to higher manufacturing costs, as it is difficult to manufacture small plastic components with the prescribed design, to assemble them and to fix them to each other by means of, for instance, welding or gluing.

In one particular embodiment the collecting chamber has a ring shape, i.e. a ring-like structure, whereat the fluids enter the collecting chamber at different points on its outer circumference. For instance, two second channels are provided, being arranged in the housing in such way that a second channel is positioned on each side of a centrally arranged first channel, whereat the second channels end in the ring-shaped collecting chamber at angle differences of +90° and -90° with respect to the end point of the first channel (0°). In this case, the exit point of the fluid or fluid mixture within the collecting chamber communicating with the outlet can be arranged opposite to the end point of the first channel, i.e. there is an angle difference of 180° between the end point of the first channel and the exit point of the collecting chamber.

Regarding the at least one valve of the device according to the invention, it can be provided that at least one section of the valve has a cylindrical shape, the radial surface of which comprises a recess extending over the circumference of the radial surface, wherein the recess provides a communication between the second channel and the indicator chamber in the first position of the valve, and between the second channel and the collecting point in the second position of the valve. By means of such a valve, the adjustment between the first and second position, and vice versa, can be carried out by turning its cylindrical section about the axial direction of the same. On the cylindrical section a grip portion can be formed, allowing for an easy manual adjustment of the valve. The cylindrical section of the valve can moreover be inserted into the housing, in which case there is a clearance formed within the housing of the device into which the cylindrical section of the valve is inserted and fixed in such a manner, that the cylindrical section is still turnable, e.g., by means of a tongue and groove connection. On the radial surface of the cylindrical section, a recess is formed that extends over the circumference of the radial surface allowing thus for an adjustable fluid connection between the second channel and the indicator chamber or the collecting point, whereat the adjustment can be conducted by turning the cylindrical section of the valve about its axial direction from the first position to the second position, or vice versa. The length of the recess formed on the radial surface of the cylindrical section depends on the angle difference between the location of the points of the second channel, which adjoin the cylindrical section and communicate with the second inlet, the collecting point or the indicator chamber.

A particular preferred embodiment of the device according to the invention is described hereinafter with reference to figures, whereat
- Fig. 1: shows a perspective view of an infusion system comprising a preferred embodiment of the device according to the present invention,
- Fig. 2: shows a sectional view of the preferred embodiment of the device,
- Fig. 3: shows a further sectional view of the preferred embodiment of the device,
- Fig. 4: shows a further sectional view of the preferred embodiment of the device,
- Fig. 5: shows a perspective view of an insertion member being one component of the preferred embodiment of the device,
- Fig. 6: shows a sectional view of the insertion member depicted in Fig. 5,
- Fig. 7: shows a perspective view of a further insertion member being one component of the preferred embodiment of the device,
- Fig. 8: shows a perspective view of a valve being one component of the preferred embodiment of the device,
- Fig. 9: shows a sectional view of the valve depicted in Fig. 8,
- Fig. 10: shows a perspective view of an insertion element being one component of the preferred embodiment of the device,
- Fig. 11: shows a sectional view of the insertion element depicted in Fig. 10,
- Fig. 12: depicts an exploded view of the preferred embodiment of the device, and
- Fig. 13: shows a chart of measurement values of flow rates over time, measured both with a device according to prior art and with the preferred embodiment of the device according to the present invention.

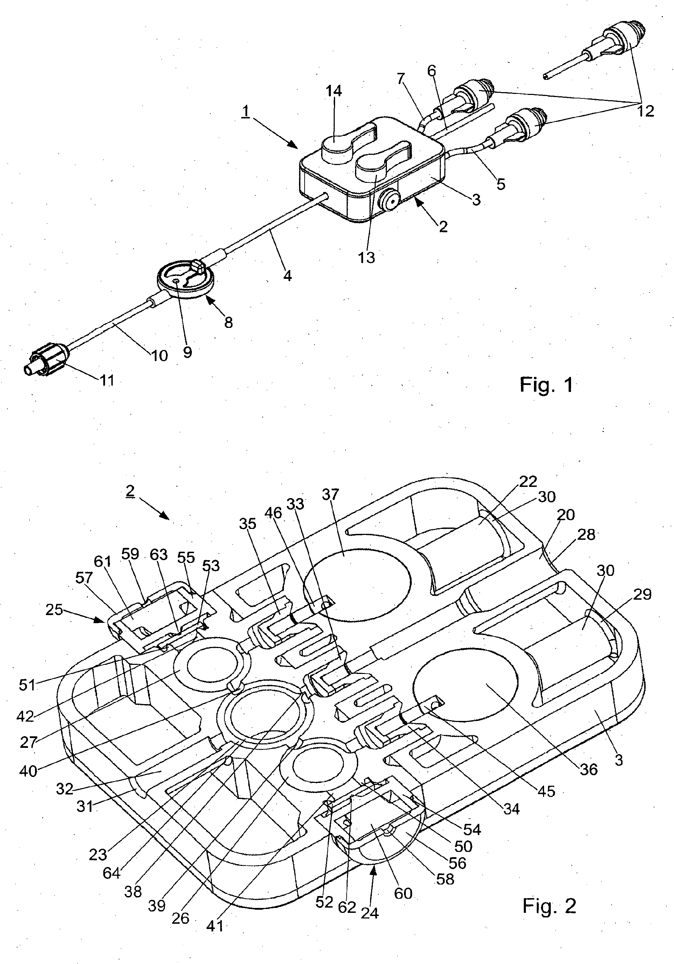

Figure 1 shows the preferred embodiment of the device 2 according to the invention in a perspective view, which is shown as a part of an infusion system 1, and in more detail, in the following figures. The device 2 comprises a housing 3 to which tubes 4, 5, 6, 7 are connected. The tube 6 leads to the first inlet of the housing 3, the tubes 5, 7 lead to a second inlet of the housing 3, respectively, and the tube 4 is connected to the outlet of the housing 3 and leads to an external filter chamber 8 enclosing a hydrophobic filter. The external filter chamber 8 has an opening 9 formed in its housing such that gas bubbles can escape through the hydrophobic filter and the opening 9. Such an external filter chamber 8 is already employed with state of the art infusion systems and is therefore not part of the present invention. The tube 10 leads to the patient. On the end portion of the tube 10, a male connector 11 of a luer-lock-connection is attached. The connector 11 is connected to an infusion needle (not shown) during the use of the infusion system 1. A female connector 12 of a luer-lock-connection is attached to the end portions of tubes 5, 6, 7, respectively, allowing for a connection of the respective tube 5, 6, 7 to a medicament source or a pump driving medicaments to the respective tubes 5, 6, 7. The device 2 comprises moreover two grip portions 13, 14, to which is made reference hereinafter.

Figure 2 shows a horizontal sectional view of the device 2. The device comprises a housing 3, in which a first channel 20, two second channels 21, 22, a collecting chamber 23, two indicator chambers 24, 25 and two valves 26, 27 are arranged. The housing 3 further comprises a first inlet 28, two second inlets 29, 30, an outlet 31 communicating with the collecting chamber 23 through an outlet channel 32, three check valves 33, 34, 35, and two filter chambers 36, 37.

The first channel 20 extends through the housing starting from the first inlet 28 and ending at the collecting chamber 23. Within the first channel 28 check valve 33 is arranged in the vicinity of the collecting chamber 23, preventing back flow from the collecting chamber 23 into the first channel 20.

The collecting chamber 23 is essentially ring-shaped. There is a protrusion 38 formed on the inner wall of the ring-shaped collecting chamber 23, which protrusion 38 extends marginally into the first channel 20. The protrusion 38 results in the fluid fed to the first channel 20 being separated into two half streams, flowing through the collecting chamber 23 in different directions, i.e. clockwise and counterclockwise, when it enters the collecting chamber 23. The end of outlet channel 32 and protrusion 38 have an angle difference of 180°. The collecting chamber 23 is realized by providing a clearance in the housing 3, into which a cylindrical-shaped insertion member 64 with a recess 65 formed on its radial surface is inserted. The collecting chamber 23 is formed by the recess of insertion member 64 and the circumferential surface of the clearance.

The second channels 21, 22 extend through the housing 3, starting from the second inlets 29, 30 and ending at the indicator chambers 24, 25, or at the collecting chamber 23. The second channels 21, 22 each have a branched structure, wherein one of the valves 26, 27 is arranged at the branching point such that an adjustable fluid connection between the second channels 21, 22 and the indicator chambers 24, 25 or between the second channels 21, 22 and the collecting chamber 23 can be provided there. In each second channel, one of the check valves 34, 35 is arranged preventing backflow from the collecting chamber 23 or the indicator chambers 24, 25 towards the second inlets 29, 30. In order to minimize the dead volume of the device 2 the branches 39, 40, 41, 42 of the second channels 21, 22 are designed to be very short and extend merely through the walls separating the valves 26, 27 from the indicator chambers 24, 25 and the walls separating the valves 26, 27 from the collecting chamber 23. The minimal thickness of these walls depend on the desired mechanical stability of the device 2. The second channels 21, 22 further comprise filter chambers 36, 37, in which a hydrophilic filter member (not shown) or, in the present embodiment, a respective hydrophobic filter member (not shown) are arranged. In the sectional view of figure 2 the outlets 45, 46 of the filter chambers 36, 37 are shown, which are covered by said hydrophilic filter members. These hydrophilic filter members extend horizontally throughout the entire respective filter chambers 36, 37 in the present embodiment, such that the latter are separated into two sub-chambers, one of which communi-cates with the upstream portion of the respective second channel 21, 22 and one of which communicates with an outlet 45, 46 of the respective filter chamber 36, 37.

Each of the valves 26, 27 comprise a cylindrical section. On the radial surface of the cylindrical sections a corresponding recess 43, 44 is formed. The recesses 43, 44 extend over the radial surface of the cylindrical sections of the valves 26, 27 in order to provide an adjustable fluid connection between the second channels 21, 22 and the indicator chambers 24, 25 (first position of the valves 26, 27) or the collecting chamber 23 (second position of the valves 26, 27), whereat the adjustment is carried out by turning the cylindrical sections about their axial directions, moving the recesses 43, 44 at the same time. The grip portions 13, 14 shown in figure 1 are formed on the cylindrical sections. Each valve 26, 27 comprises therefore one of the grip portions 13, 14 and one cylindrical section extending into the housing 3, whereat the cylindrical sections extend vertically into the housing 3 in the present embodiment. Clearances for receiving the cylindrical sections of the valves 26, 27 are provided in the housing 3.

Indicator chambers 24, 25 communicate with the second channels 21, 22 in the first position of the valves 26, 27, which is the starting adjustment during employment of the device 2. The indicator chambers 24, 25 comprise each an inlet opening 50, 51, a sealing element 52, 53, an insertion element 54, 55, a wall element 56, 57 with an opening 58, 59 and an indicator element 60, 61. The inlet openings 50, 51 have a protruded edge against which the sealing elements 52, 53 rest. Sealing elements 52, 53 are supported by protrusions 62, 63 formed on the insertions elements 54, 55. Protrusions 62, 63 exert an initial tension on sealing elements 52, 53 such that they seal inlet openings 50, 51 in a pressure-less state. The sealing elements 52, 53 can be fixed on the protrusions 62, 63, by welding, gluing or the like, in order to simplify the assembly of the device 2 during manufacturing. The sealing elements 52, 53 act as check valves, as their outer edges can only be raised in the inflow direction.

The insertion elements 54, 55 are of cylindrical shape. They are each inserted into a corresponding clearance provided on the housing 3 and fixed on the circumferential surfaces of the clearance by, for instance, a frictional connection, tongue and groove connection, welding or gluing. The wall elements 56, 57 are adapted to the cross sectional shape of the insertion elements 54, 55 and are fixed on the wall elements 56, 57. They, furthermore, are transparent or translucent for visible light. The insertion elements 54, 55 receive indicator elements 60, 61 which consist in the present embodiment of sintered polyethylene (PEL). Indicator elements 60, 61 abut on the wall elements 56, 57 and cover the openings 58, 59. As mentioned hereinbefore, sintered polyethylene is a porous plastic material such that air can pass through it. However, if the material is in contact with a fluid, it gets densified to an impervious state. Therefore, the openings 58, 59 are sealed by indicator element 60, 61, if a fluid enters the indicator chambers 24, 25 and gets in contact with indicator elements 60, 61. So, the air located within the second channels 21, 22 before employment of the device 2 is driven to indicator chambers 24, 25 by the fluids entering the second inlets 29, 30 and escapes via the openings 58, 59, provided that the valves 26, 27 are in their first positions. When the fluids reach the indicator elements 60, 61, the latter are densified and seal openings 58, 59.

Indicator elements 60, 61 furthermore feature the property of a change in colour in case of fluid contact, which property can be achieved by additives. This allows for an easy observation whether a fluid has reached not only the respective indicator chamber 24, 25, but the indicator element 60, 61 as well, i.e. that the air contained in the second channel 21, 22 upstream the indicator element 60, 61 was removed, and that the second channel 21, 22 and the recess 43, 44 of the valve 26, 27 are filled with the fluid fed to the respective second inlet 29, 30. If administration of the fluid fed to one of the second inlets 29, 30 is to be started, the respective valve 26, 27 is switched from its first position to its second position, in which the respective second channel 21, 22 communicates with the collecting chamber 23. Switching of both valves 26, 27 can occur at the same time or at different points in time, depending on the administration schedule set for a patient. Due to the fact, that the respective second channel 21, 22 and the respective recess 43, 44 are already filled with fluid, the delay between switching of the valve 26, 27 and administration of the fluid to a patient is shortened, which allows for a more precise dosage. In the second positions of the valves 26, 27, the fluids fed to the second inlets 29, 30 flow to the collecting chamber 23 via recesses 43, 44 and enter the collecting chamber 23 at locations with an angle difference with respect the protrusion 38 and thus, with respect to the end portion of the first channel 20, of ± 90°. The fluids fed to the second inlet 29, 30 converge with the fluid fed to the first inlet 28 at these locations. The mixture of said fluids flows from there to the outlet channel 32 and to the outlet 31 of the device 2.

The check valves 33, 34, 35 comprise each a cylindrical body with a protruding edge on one side and an elastic cover being fixed on the other side and having a slit formed therein. The elastic cover is formed such that it is openable by a fluid flowing in the inflow direction and closable by fluid flowing in the outflow direction. The protruding edge rests against a corresponding contact surface of the housing 3 to which the protruding edge can be fixed, e.g., by means of welding or gluing.

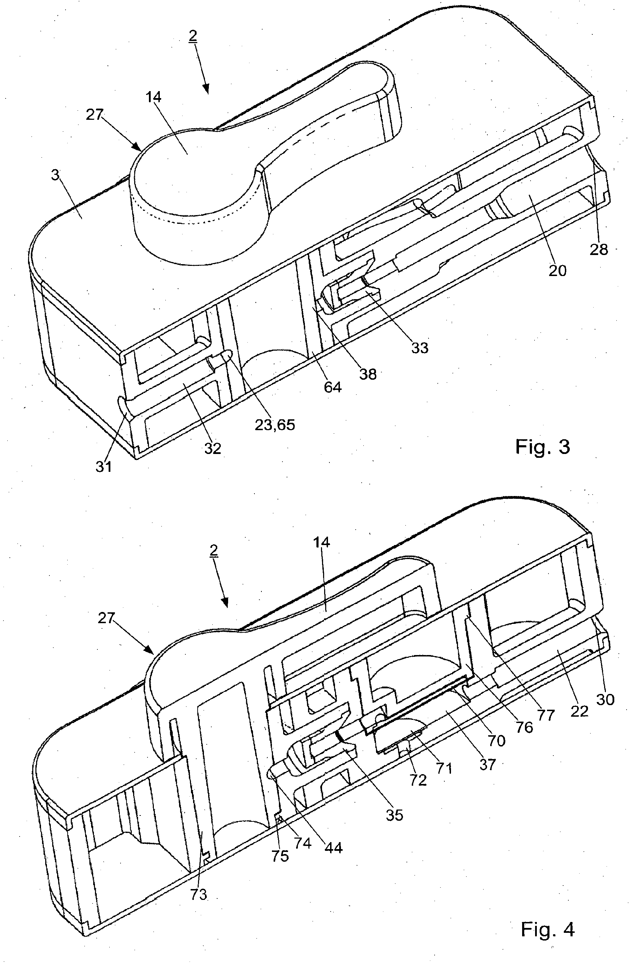

Figure 3 shows a sectional view of the present embodiment of the device 2, whereat one half of a vertical section through the first channel 20 is shown. A check valve 33 is arranged within a first channel 20 in the vicinity of collecting chamber 23. The collecting chamber 23 is formed by the circumferential surface of a clearance provided in the housing 3 and a recess 65 on the radial surface of an insertion member 64. The insertion member 64 is inserted into said clearance and fixed there by means of, for instance, a frictional connection, welding or gluing. Furthermore, figure 3 shows an outlet 31, an outlet channel 32 communicating with the collecting chamber 23 and a valve 27 with a grip portion 14. Moreover, it is shown that the device 2 has a hollow structure, while the channels and sections necessary for the intended functionality are formed by walls of a particular thickness and the rest of the housing 3 is hollow.

Figure 3 shows a sectional view of the present embodiment of the device 2, whereat the other half of a vertical section through the first channel 20 is shown. A second channel 22 leads at first from a second inlet 30 to a filter chamber 37. Within the filter chamber 37, a hydrophilic filter member 70 and a hydrophobic filter member 71 are arranged, whereat hydrophilic filter member 70 extends horizontally throughout the entire filter chamber 37 separating thus the filter chamber 37 into two sub-chambers. The filter member 70 is welded or glued with its outer edge to a corresponding contact surface of the housing. The hydrophobic filter member 71 covers an opening 72 arranged in the housing 3, and is fixed on the surface of the filter chamber 37 that surrounds the opening 72 by, for example, welding or gluing. The opening 72 communicates with the ambience air of the device 2. The hydrophobic filter 71 and the opening 72 allow gas bubbles to escape from the fluid fed through second channel 22. Moreover, air contained within second channels 22 upstream the filter chamber 37 can escape through the hydrophobic filter member 71 and the opening 72. The filter chamber 37 is delimited partly by an insertion member 76, which is essentially cylindrical-shaped. An insertion member 76 has a protruding edge 77 on its one side, which allows for a defined positioning of the insertion member 76 during the assembly of the device and, moreover, which provides a contact surface for the fixation of the insertion member 76 on the housing 3, for instance, by welding or gluing.

The upper sub-chamber of the filter chamber 37 communicates with the downstream portion of the second channel 22 via an outlet 46, which is formed on the insertion member 76. Within the downstream portion of the second channel 22, a check valve 35 is arranged. Downstream the check valve 35, the second channel 22 leads to a recess 44 located on the radial surface of the cylindrical section 73 of the valve 27. The valve 27 is fixed on the housing by means of a tongue 74 and groove 75 connection, whereat the groove 75 is formed on the radial surface of the cylindrical section 73. By means of the grip portion 14, the valve 27 is manually turnable in an easy manner. The lower sub-chamber of the filter chamber 37 communicates with the second inlet 30.

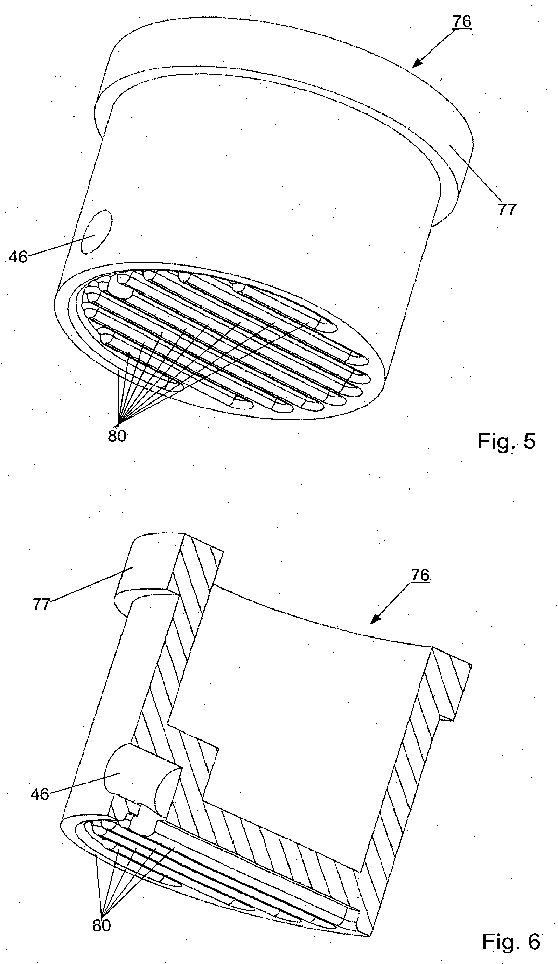

Figure 5 shows the insertion member 76. The insertion member 76 has a substantially cylindrical shape and comprises a protruding edge 77 on one side and protrusions 80 on the frontal surface of the other side. The protrusions 80 support the hydrophilic filter member 70 and can be fixed on it, e.g., by welding or gluing. Furthermore, the outlet 46 is formed on the insertion member 76.

Figure 6 shows a vertical section view of the insertion member 76. Protrusions 80 are formed on its frontal surface, and a protruding edge 77 on its other side. The outlet 46 extends from said frontal surface to the cylindrical-shaped radial surface of the insertion member 76.

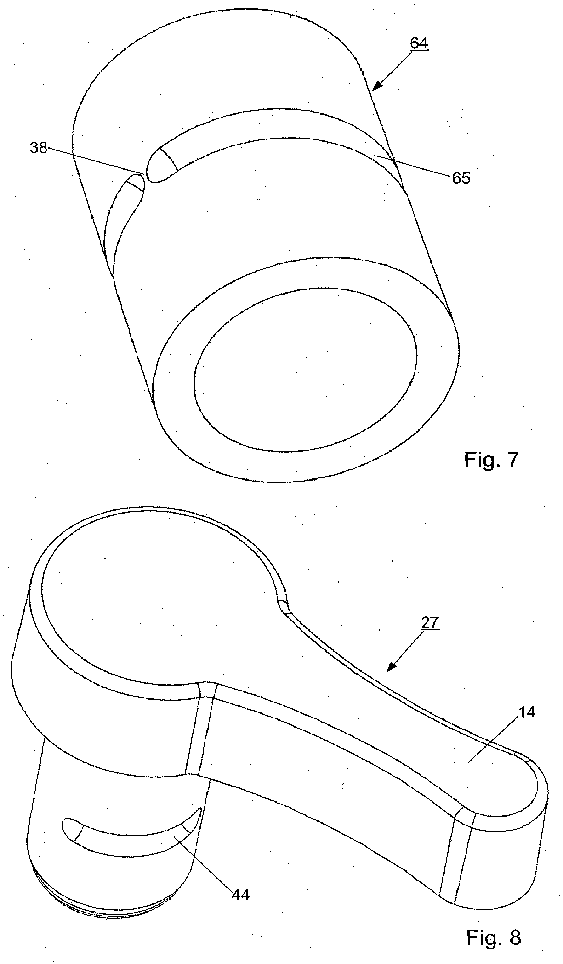

Figure 7 shows the insertion member 64 by means of which the collecting chamber 23 is realized. The insertion member 64 has a cylindrical shape, whereat on its radial surface a recess 65 is formed, which extends almost along its entire circumference. Between the two ends of the recess 65, a protrusion 38 is located and serves the purpose of splitting the fluid flow fed to the first channel 20, as previously described. The insertion member 64 is inserted into a corresponding clearance of the housing 3 during the assembly of device 2.

Figure 8 depicts valve 27 in a perspective view. The structure of valve 27 is mirrored symmetrically to the structure of valve 26, in case the grip portions 13, 14 are oriented parallely in their first and second positions. Valve 27 comprises a cylindrical section with a recess 44 on its radial surface. Recess 44 provides a fluid connection between the second channel 22 and the indicator chamber 25 or collecting chamber 23, depending on whether valve 27 is in its first position or its second position. Adjustment between both positions can be carried out by turning the grip portion 14 about the axial direction of the cylindrical section and changing its orientation by 90°.

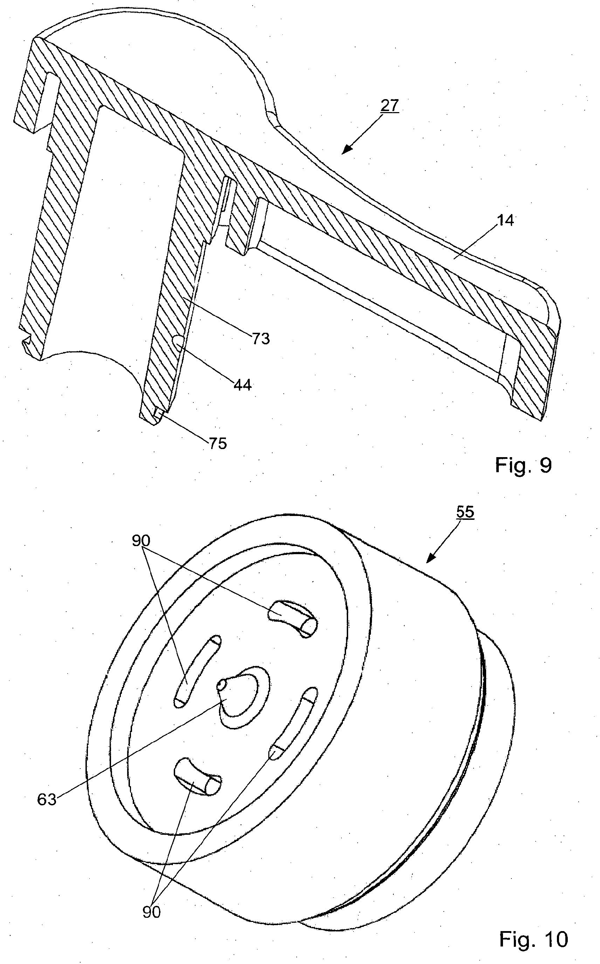

Figure 9 shows valve 27 in a vertical section view. The grip portion 14, a part of the recess 44 and the groove 75 are shown. By means of the groove 75 and the corresponding tongue 74, the valve 27 is turnably and imperviously fixed on the housing 3.

Figure 10 shows the insertion element 55 of the indicator chamber 25, which is identical to the insertion element 54 of the indicator chamber 24, in a perspective view. It is of cylindrical shape and comprises a protrusion 63 and openings 90, through which the fluid flowing into the indicator chamber 25 passes the insertion element 55 and reaches indicator element 61. The sealing element 53 rests against the protrusion 63 such that only the outer edge of the sealing element 53 is raisable in order to allow fluid to enter the collecting chamber 25.

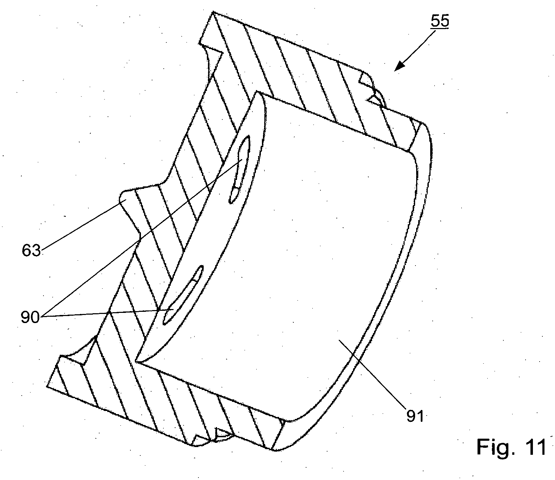

Figure 11 shows the insertion element 55 in a vertical section view. Openings 90 and a protrusion 63 are shown. The inner space 91 of the insertion element 55 receives the indicator element 61, and can additionally receive a separate hydrophobic filter element.

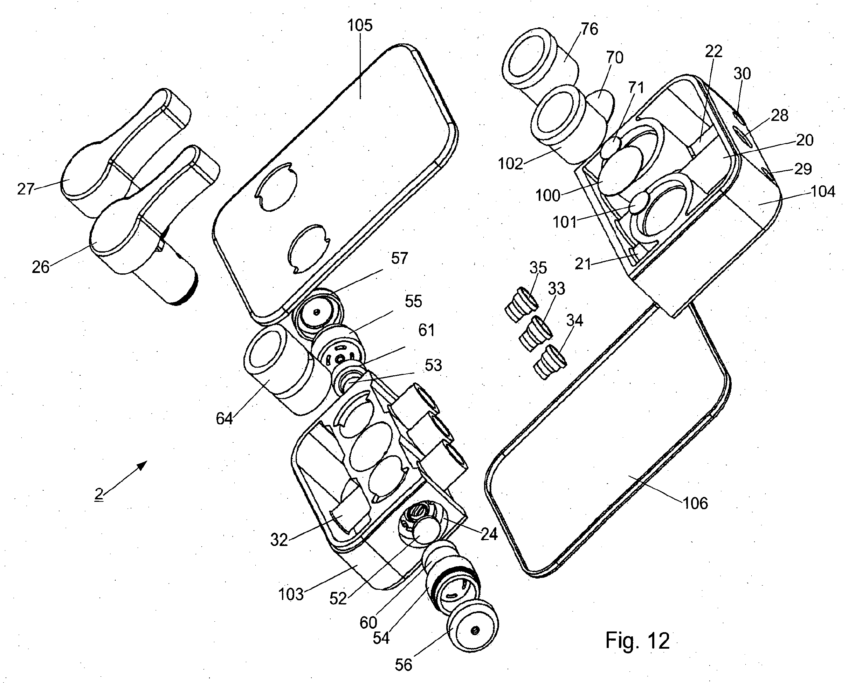

Figure 12 shows an exploded perspective view of the present embodiment of device 2. Next to the components described above, a hydrophilic filter member 100, a hydrophobic filter member 101, an insertion member 102 and housing elements 103, 104, 105, 106 are shown. The filter members 100, 101 are the equivalents of filter members 70, 71 and are arranged in the filter chamber 36 comprised by the second channel 21. The insertion member 102 is the equivalent of the insertion member 76. Housing elements 103, 104, 105, 106 constitute the housing 3. The housing elements 103, 104, 105, 106 are designed such that they are as few as possible in number and allow for an easy assembly of all components of the device 2. When assembling the device 2, check valves 33, 34, 35 are arranged in their final positions and fixed on the housing element 103 with their protruding edges by, for example, a frictional connection, a tongue and groove connection, welding, in particular ultra sonic welding, or gluing. Then, the housing element 103 and the housing element 104 are plugged together and fixed to one another by, for example, a frictional connection, a tongue and groove connection, welding, in particular ultra sonic welding, or gluing. Then, all other components, except valves 26, 27 and housing element 104, are arranged in their respective final positions and fixed there by, for example, a frictional connection, a tongue and groove connection, welding, in particular ultra sonic welding, or gluing. Then, the housing element 105 is fixed on housing elements 103, 104, by, for example, a frictional connection, a tongue and groove connection, welding, in particular ultra sonic welding, or gluing. Finally, the valves 26, 27 are inserted into the corresponding clearances of housing elements 103, 105 and fixed there by means of the said tongue and groove connection (see description of figures 4 and 9).

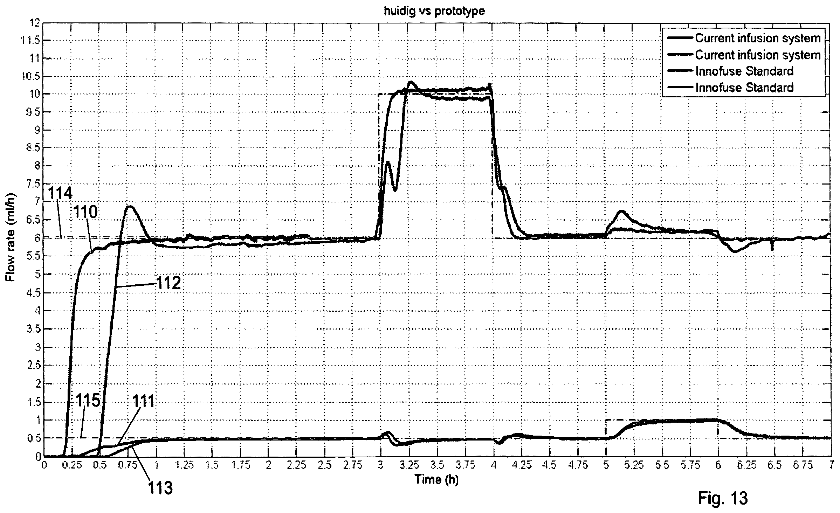

Figure 13 shows a chart of measurement values of flow rates 110, 111 taken with the preferred embodiment of device 2 as shown in figures 1 to 12, and measurement values of flow rates 112, 113 taken with an infusion system according to prior art, by means of which several fluids converge and are fed to a patient as mixture through a single tube. The dashed lines 114, 115 show the pump settings of pumps that drive the fluids through the first 20 and one of the second 21, 22 channels. One pump is allocated to each tube leading to device 2, so as to independently adjust the flow rates. The dashed line 114 shows the pump setting of the pump connected to the first inlet 28, whereas the dashed line 115 shows the setting of the pump connected to one of the second inlets 29, 30. A sodium chloride solution or nutritive solution will generally be fed through the first channel 20, whereas medicaments will normally be fed through the second channels 21, 22. Therefore, the flow rate of the fluid fed through the first channel 20 usually amounts to between 6 ml/h and 12 ml/h, whereas the flow rate of the fluids fed through the second channels 21, 22, amounts to between 0.5 ml/h and 2 ml/h. The pressure within the fluid guiding parts of the device 2 and the tubes leading to it typically amounts to 2 bar to 3 bar.

Referring to figure 13, there are three effects observable with prior art infusion systems. First, the delay between activation of the pumps occurring at 0 on the x-axis of figure 13 and administration of the fluids, i.e. the point in time at which the fluids have reached at least the connector 11 (figure 1). The delay is noticeably shorter with the device according to the invention. This is due to the fact that the infusion system 1 comprising the device 2 is already filled up to the valves 26, 27, including the recesses 43, 44. If the administration of a medicament is to be initiated, the corresponding valve is turned from its first position to its second position, whereat the remaining length of the flow path, i.e. the length of the flow path between valves 26, 27 and the connector 11, is shorter. So, the reference time serving as a basis for the dose calculation is closer to the point in time at which administration actually starts. The presence of fluids in the indicator chambers 24, 25 is easily observable thanks to the indicator elements 60,61 changing their colour in case of fluid contact, and the wall elements 56,57 being transparent or translucent for visible light.

A second effect observable with prior art infusion systems is the so-called over shoot occurring with the flow rate 112 between 0.5 h and 1 h and between 3.25 h and 3.5 h. This problem is remedied with the device 2, as can be seen on the course of flow rate 110.

A third effect observable with prior art infusion systems are flow rate fluctuations occurring if one of two or more flow rates is set to another value by means of a pump. Such fluctuations occur with the flow rate 112 between 5.0 h and 5.25 h, where the pump setting is set from 0.5 ml/h to 1.0 ml/h, and between 6.0 h and 6.25 h, where the pump setting 115 is set from 1.0 ml/h to 0.5 ml/h. With the flow rate 113 such fluctuations occur between 3.0 h and 3.25 h, where the pump setting 114 is set from 6.0 ml/h to 10 ml/h, and between 4.0 and 4.25 h, where the pump setting 114 is set from 10 ml/h to 6 ml/h. These fluctuations are remarkably minimized with the device 2, as can be seen on the course of flow rates 110, 111.

The courses of flow rates 110, 111 are in general remarkably closer to the courses of the respective pump settings 114, 115, i.e. a more precise dosage is achieved.

While the invention has been described with reference to a specific embodiment, the description is merely explanatory and is not to be construed as limiting the scope of the invention. Various modifications may be conceived by those skilled in the art without departing from the scope of the invention as defined by the claims.

### List of references

- 1: infusion system
- 2: device
- 3: housing
- 4: tube
- 5: tube
- 6: tube
- 7: tube
- 8: external filter chamber
- 9: opening
- 10: tube
- 11: male connector
- 12: female connector
- 13: grip portion
- 14: grip portion
- 20: first channel
- 21: second channel
- 22: second channel
- 23: collecting chamber
- 24: indicator chamber
- 25: indicator chamber
- 26: valve
- 27: valve
- 28: first inlet
- 29: second inlet
- 30: second inlet
- 31: outlet
- 32: outlet channel
- 33: check valve
- 34: check valve
- 35: check valve
- 36: filter chamber
- 37: filter chamber
- 38: protrusion
- 39: branch
- 40: branch
- 41: branch
- 42: branch
- 43: recess
- 44: recess
- 45: outlet
- 46: outlet
- 50: inlet opening
- 51: inlet opening
- 52: sealing element
- 53: sealing element
- 54: insertion element
- 55: insertion element
- 56: wall element
- 57: wall element
- 58: opening
- 59: opening
- 60: indicator element
- 61: indicator element
- 62: protrusion
- 63: protrusion
- 64: insertion member
- 65: recess
- 70: hydrophilic filter member
- 71: hydrophobic filter member
- 72: opening
- 73: cylindrical section
- 74: tongue
- 75: groove
- 76: insertion member
- 77: protruding edge
- 80: protrusions
- 90: openings
- 91: inner space
- 100: hydrophilic filter member
- 101: hydrophobic filter member
- 102: insertion member
- 103: housing element
- 104: housing element
- 105: housing element
- 106: housing element
- 110: flow rate over time
- 111: flow rate over time
- 112: flow rate over time
- 113: flow rate over time
- 114: pump setting over time
- 115: pump setting over time

## Claims

1. Device (2) for the administration of fluids to a human or animal body, comprising a housing (3) with a first inlet (28), at least one second inlet (29, 30), one outlet (31) and a collecting point, wherein the first inlet (28) communicates with the collecting point through a first channel and the outlet (31) communicates with the collecting point,
**characterized in that**
the second inlet (29, 30) communicates with the collecting point or an indicator chamber (24, 25) through a second channel (21, 22) in which a valve (26, 27) is arranged, the valve (26, 27) being adjustable between a first position and a second position, wherein, in the first position of the valve (26, 27), the second channel (21, 22) communicates with the indicator chamber (24, 25), and, in the second position of the valve (26, 27), the second channel (21, 22) communicates with the collecting point and the indicator chamber (24, 25) comprises an indicator element (60, 61) which changes its color in case of fluid contact.

2. Device (2) according to claim 1,
**characterized in that**
the indicator chamber (26, 27) and/or the housing (3) is at least in part transparent or translucent for visible electromagnetic radiation.

3. Device (2) according to claim 1 or 2,
**characterized in that**
the indicator element (60, 61) is made of a sintered plastic material.

4. Device (2) according to claim 3,
**characterized in that**
the plastic material comprises at least one additive which changes its color in case of fluid contact.

5. Device (2) according to claim 3 or 4,
**characterized in that**
the plastic material is polyethylene.

6. Device (2) according to claim 2,
**characterized in that**
the hydrophobic filter element or the indicator element (60, 61) abuts on a wall of the indicator chamber (24, 25) with at least one opening (58, 59) communicating with the ambience air of the device (2).

7. Device (2) according to claim 1,
**characterized in that**
an inlet opening (50, 51) of the indicator chamber (24, 25) is covered by an elastic sealing element (52, 53) acting as a check valve which is closed in a pressure-less state.

8. Device (2) according to claim 2 or 7,
**characterized in that**
the indicator chamber (24, 25) comprises at least one insertion element (54, 55) which holds the sealing element (52, 53) in its position by contacting the sealing element (52, 53) and/or which receives the hydrophobic filter element and/or the indicator element (60, 61).

9. Device (2) according to claim 6 and 8,
**characterized in that**
the wall of the indicator chamber (24, 25) with at least one opening (58, 59) is embodied as a wall element (56, 57), which is fixed on the insertion element (54, 55).

10. Device (2) according to claim 1,
**characterized in that**
an indicator mechanism is arranged within the indicator chamber, comprising at least one movable part which moves with respect to the indicator chamber when the indicator chamber is filled with a fluid.

11. Device (2) according to claim 10,
**characterized in that**
the movable part has a color mark.

12. Device (2) according to claim 1,
**characterized in that**
the second channel (21, 22) and/or the first channel (20) comprises a filter chamber (36, 37) in which a hydrophilic filter member (70, 100) and/or a hydrophobic filter member (71, 101) is arranged, wherein the hydrophilic filter member (70, 100) separates the filter chamber (36, 37) into to sub-chambers and wherein the hydrophobic filter member (71, 101) covers an opening (72) of the filter chamber (36, 37) which is in communication with the ambience air of the device.

13. Device (2) according to claim 12,
**characterized in that**
the hydrophilic filter member (70, 100) is supported by protrusions (80) extending from at least one wall of the filter chamber (36, 37).

14. Device (2) according to claim 1,
**characterized in that**
a check valve (33, 34, 35) is arranged within the first channel (20) and/or within the second channel (21, 22).

15. Device (2) according to claim 1,
**characterized in that**
the collecting point is embodied as a collecting chamber (23).

16. Device (2) according to claim 15,
**characterized in that**
the collecting chamber (23) is ring- shaped.

17. Device (2) according to claim 1,
**characterized in that**
at least one section (73) of the valve (26, 27) has a cylindrical shape, the radial surface of which comprises a recess (43, 44) extending over the circumference of the radial surface, wherein the recess (43, 44) provides a communication between the second channel (21, 22) and the indicator chamber (24, 25) in the first position of the valve (26, 27), and between the second channel (21, 22) and the collecting point in the second position of the valve (26, 27).

## Patentansprüche

1. Vorrichtung (2) zur Verabreichung von Fluiden an einen menschlichen oder tierischen Körper, umfassend ein Gehäuse (3) mit einem ersten Einlass (28), mindestens einem zweiten Einlass (29, 30), einem Auslass (31) und einem Sammelpunkt, wobei der erste Einlass (28) durch einen ersten Kanal mit dem Sammelpunkt in Verbindung steht und der Auslass (31) mit dem Sammelpunkt in Verbindung steht,
**dadurch gekennzeichnet,**
**dass** der zweite Einlass (29, 30) durch einen zweiten Kanal (21, 22), in dem ein Ventil (26, 27) angeordnet ist, mit dem Sammelpunkt oder einer Anzeigekammer (24, 25) in Verbindung steht, wobei das Ventil (26, 27) zwischen einer ersten Position und einer zweiten Position einstellbar ist, wobei der zweite Kanal (21, 22) in der ersten Position des Ventils (26, 27) mit der Anzeigekammer (24, 25) in Verbindung steht und der zweite Kanal (21, 22) in der zweiten Position des Ventils (26, 27) mit dem Sammelpunkt in Verbindung steht und die Anzeigekammer (24, 25) ein Anzeigeelement (60, 61) umfasst, das bei Kontakt mit Fluid seine Farbe ändert.

2. Vorrichtung (2) gemäß Anspruch 1,
**dadurch gekennzeichnet,**
**dass** die Anzeigekammer (26, 27) und/oder das Gehäuse (3) zumindest teilweise für sichtbare elektromagnetische Strahlung transparent oder durchlässig ist.

3. Vorrichtung (2) gemäß Anspruch 1 oder 2,
**dadurch gekennzeichnet,**
**dass** das Anzeigeelement (60, 61) aus einem gesinterten Kunststoffmaterial gefertigt ist.

4. Vorrichtung (2) gemäß Anspruch 3,
**dadurch gekennzeichnet,**
**dass** das Kunststoffmaterial mindestens einen Zusatzstoff umfasst, der bei Kontakt mit Fluid seine Farbe ändert.

5. Vorrichtung (2) gemäß Anspruch 3 oder 4,
**dadurch gekennzeichnet,**
**dass** das Kunststoffmaterial Polyethylen ist.

6. Vorrichtung (2) gemäß Anspruch 2,
**dadurch gekennzeichnet,**
**dass** das hydrophobe Filterelement oder das Anzeigeelement (60, 61) mit mindestens einer Öffnung (58, 59), die mit der Umgebungsluft der Vorrichtung (2) in Verbindung steht, an einer Wand der Anzeigekammer (24, 25) anliegt.

7. Vorrichtung (2) gemäß Anspruch 1,
**dadurch gekennzeichnet,**
**dass** eine Einlassöffnung (50, 51) der Anzeigekammer (24, 25) von einem elastischen Dichtungselement (52, 53) bedeckt ist, das als Rückschlagventil dient, das in einem drucklosen Zustand geschlossen ist.

8. Vorrichtung (2) gemäß Anspruch 2 oder 7,
**dadurch gekennzeichnet,**
**dass** die Anzeigekammer (24, 25) mindestens ein Einführelement (54, 55) umfasst, welches das Dichtungselement (52, 53) durch Kontaktieren des Dichtungselements (52, 53) in seiner Position hält und/oder welches das hydrophobe Filterelement und/oder das Anzeigeelement (60, 61) aufnimmt.

9. Vorrichtung (2) gemäß Anspruch 6 und 8,
**dadurch gekennzeichnet,**
**dass** die Wand der Anzeigekammer (24, 25) mit mindestens einer Öffnung (58, 59) als Wandelement (56, 57) ausgebildet ist, das auf dem Einführelement (54, 55) befestigt ist.

10. Vorrichtung (2) gemäß Anspruch 1,
**dadurch gekennzeichnet,**
**dass** ein Anzeigemechanismus innerhalb der Anzeigekammer angeordnet ist, umfassend mindestens ein bewegliches Teil, das sich relativ zur Anzeigekammer bewegt, wenn die Anzeigekammer mit einem Fluid gefüllt ist.

11. Vorrichtung (2) gemäß Anspruch 10,
**dadurch gekennzeichnet,**
**dass** das bewegliche Teil eine Farbmarkierung hat.

12. Vorrichtung (2) gemäß Anspruch 1,
**dadurch gekennzeichnet,**
**dass** der zweite Kanal (21, 22) und/oder der erste Kanal (20) eine Filterkammer (36, 37) umfasst, in der ein hydrophiles Filterglied (70, 100) und/oder ein hydrophobes Filterglied (71, 101) angeordnet ist, wobei das hydrophile Filterglied (70, 100) die Filterkammer (36, 37) in Unterkammern teilt und das hydrophobe Filterglied (71, 101) eine Öffnung (72) der Filterkammer (36, 37) bedeckt, die mit der Umgebungsluft der Vorrichtung in Verbindung steht.

13. Vorrichtung (2) gemäß Anspruch 12,
**dadurch gekennzeichnet,**
**dass** das hydrophile Filterglied (70, 100) durch Vorsprünge (80) getragen wird, die sich von mindestens einer Wand der Filterkammer (36, 37) erstrecken.

14. Vorrichtung (2) gemäß Anspruch 1,
**dadurch gekennzeichnet,**
**dass** ein Rückschlagventil (33, 34, 35) innerhalb des ersten Kanals (20) und/oder innerhalb des zweiten Kanals (21, 22) angeordnet ist.

15. Vorrichtung (2) gemäß Anspruch 1,
**dadurch gekennzeichnet,**
**dass** der Sammelpunkt als Sammelkammer (23) ausgebildet ist.

16. Vorrichtung (2) gemäß Anspruch 15,
**dadurch gekennzeichnet,**
**dass** die Sammelkammer (23) ringförmig ist.

17. Vorrichtung (2) gemäß Anspruch 1,
**dadurch gekennzeichnet,**
**dass** mindestens ein Abschnitt (73) des Ventils (26, 27) eine zylindrische Form aufweist, deren radiale Oberfläche eine Ausnehmung (43, 44) umfasst, die sich über den Umfang der radialen Oberfläche erstreckt, wobei die Ausnehmung (43, 44) in der ersten Position des Ventils (26, 27) eine Verbindung zwischen dem zweiten Kanal (21, 22) und der Anzeigekammer (24, 25), und in der zweiten Position des Ventils (26, 27) eine Verbindung zwischen dem zweiten Kanal (21, 22) und dem Sammelpunkt bereitstellt.

## Revendications

1. Dispositif (2) d'administration de fluides à un corps humain ou d'animal, comprenant un boîtier (3) possédant une première entrée (28), au moins une seconde entrée (29, 30), une sortie (31) et un point de collecte, dans lequel la première entrée (28) communique avec le point de collecte par l'intermédiaire d'un premier canal et la sortie (31) communique avec le point de collecte,
**caractérisé en ce**
**que** la seconde entrée (29, 30) communique avec le point de collecte ou une chambre indicatrice (24, 25) par l'intermédiaire d'un second canal (21, 22) dans lequel est disposée une soupape (26, 27), la soupape (26, 27) étant réglable entre une première position et une seconde position, dans lequel, dans la première position de la soupape (26, 27), le second canal (21, 22) communique avec la chambre indicatrice (24, 25), et, dans la seconde la position de la soupape (26, 27), le second canal (21, 22) communique avec le point de collecte et la chambre indicatrice (24, 25) comprend un élément indicateur (60, 61) qui change de couleur en cas de contact avec un fluide.

2. Dispositif (2) selon la revendication 1,
**caractérisé en ce**
**que** la chambre indicatrice (26, 27) et/ou le boîtier (3) sont au moins en partie transparents ou translucides pour un rayonnement électromagnétique visible.

3. Dispositif (2) selon la revendication 1 ou 2,
**caractérisé en ce**
**que** l'élément indicateur (60, 61) est constitué d'une matière plastique frittée.

4. Dispositif (2) selon la revendication 3,
**caractérisé en ce**
**que** la matière plastique comprend au moins un additif qui change de couleur en cas de contact avec un fluide.

5. Dispositif (2) selon la revendication 3 ou 4,
**caractérisé en ce**
**que** la matière plastique est du polyéthylène.

6. Dispositif (2) selon la revendication 2,
**caractérisé en ce**
**que** l'élément de filtre hydrophobe ou l'élément indicateur (60, 61) vient buter contre une paroi de la chambre indicatrice (24, 25) avec au moins une ouverture (58, 59) communiquant avec l'air ambiant du dispositif (2).

7. Dispositif (2) selon la revendication 1,
**caractérisé en ce**
**qu'**une ouverture d'entrée (50, 51) de la chambre indicatrice (24, 25) est recouverte d'un élément d'étanchéité élastique (52, 53) faisant office de clapet anti-retour fermé à l'état sans pression.

8. Dispositif (2) selon la revendication 2 ou 7,
**caractérisé en ce**
**que** la chambre indicatrice (24, 25) comprend au moins un élément d'insertion (54, 55) qui maintient l'élément d'étanchéité (52, 53) dans sa position en venant en contact avec l'élément d'étanchéité (52, 53) et/ou qui reçoit l'élément de filtre hydrophobe et/ou l'élément indicateur (60, 61).

9. Dispositif (2) selon la revendication 6 ou 8,
**caractérisé en ce**
**que** la paroi de la chambre indicatrice (24, 25) avec au moins une ouverture (58, 59) est réalisée sous la forme d'un élément de paroi (56, 57), qui est fixé sur l'élément d'insertion (54, 55).

10. Dispositif (2) selon la revendication 1,
**caractérisé en ce**
**qu'**un mécanisme indicateur est agencé à l'intérieur de la chambre indicatrice, comprenant au moins une partie mobile qui se déplace par rapport à la chambre indicatrice lorsque la chambre indicatrice est remplie d'un fluide.

11. Dispositif (2) selon la revendication 10,
**caractérisé en ce**
**que** la partie mobile a une marque de couleur.

12. Dispositif (2) selon la revendication 1,
**caractérisé en ce**
**que** le second canal (21, 22) et/ou le premier canal (20) comprennent une chambre de filtre (36, 37) dans laquelle un élément de filtre hydrophile (70, 100) et/ou un élément de filtre hydrophobe (71, 101) est agencé, dans lequel l'élément de filtre hydrophile (70, 100) sépare la chambre de filtre (36, 37) en deux sous-chambres, et dans lequel l'élément de filtre hydrophobe (71, 101) recouvre une ouverture (72) de la chambre de filtre (36, 37) qui est en communication avec l'air ambiant du dispositif.

13. Dispositif (2) selon la revendication 12,
**caractérisé en ce**
**que** l'élément de filtre hydrophile (70, 100) est supporté par des protubérances (80) s'étendant à partir d'au moins une paroi de la chambre de filtre (36, 37).

14. Dispositif (2) selon la revendication 1,
**caractérisé en ce**
**qu'**un clapet anti-retour (33, 34, 35) est agencé à l'intérieur du premier canal (20) et/ou à l'intérieur du second canal (21, 22).

15. Dispositif (2) selon la revendication 1,
**caractérisé en ce**
**que** le point de collecte est réalisé sous la forme d'une chambre de collecte (23).

16. Dispositif (2) selon la revendication 15,
**caractérisé en ce**
**que** la chambre de collecte (23) est en forme d'anneau.

17. Dispositif (2) selon la revendication 1,
**caractérisé en ce**
**qu'**au moins une section (73) de la soupape (26, 27) a une forme cylindrique dont la surface radiale comprend un évidement (43, 44) s'étendant sur la circonférence de la surface radiale, dans lequel l'évidement (43, 44) fournit une communication entre le second canal (21, 22) et la chambre indicatrice (24, 25) dans la première position de la soupape (26, 27), et entre le second canal (21, 22) et le point de collecte dans la seconde position de la soupape (26, 27).
